# EUROPÄISCHE PATENTSCHRIFT

(11) **EP 0 699 434 B1**
(45) Veröffentlichungstag und Bekanntmachung des Hinweises auf die Patenterteilung: **24.03.1999**
(21) Anmeldenummer: 95109978.7
(22) Anmeldetag: 27.06.1995
(51) Int. Cl.: A61K 7/50, A61K 7/48, A61K 7/06

(54) **Flüssige Haut- und Körperpflegeprodukte**
Liquid personal skin- and body care product
Produits liquides pour les soins de la peau et du corps

(30) Priorität: 05.07.1994 DE 4423579; 29.05.1995 DE 19519580
(43) Veröffentlichungstag der Anmeldung: 06.03.1996
(73) Patentinhaber: Doetsch, Grether & Cie AG, 4002 Basel (CH)
(72) Erfinder: Bohli, Erich, Dr., CH-8050 Zürich (CH); Schröder, Jürgen, Dr. rer. nat. Dipl.-Chem., CH-6018 Buttisholz (CH)
(74) Vertreter: EGLI-EUROPEAN PATENT ATTORNEYS

(56) Entgegenhaltungen:
- FR-A- 1 145 887
- FR-A- 2 393 570
- FR-A- 2 688 133
- US-A- 5 126 135
- DATABASE WPI Week 9004 Derwent Publications Ltd., London, GB; AN 90028689 & SU-A-1 472 068 (AEROLOL TRUST) , 15.April 1989
- PATENT ABSTRACTS OF JAPAN vol. 12 no. 143 (C-492) [2990] ,30.April 1988 & JP-A-62 258312 (TATSUO OKAZAKI) 10.November 1987,
- CHEMICAL ABSTRACTS, vol. 116, no. 10, 9.März 1992 Columbus, Ohio, US; abstract no. 91166m, Seite 490; & JP-A-03 291 215 (TAMURA KAGAKU K.K.) 20.Dezember 1991

## Beschreibung

Die Erfindung betrifft flüssige Haut- und Körperpflegeprodukte, insbesondere wässerige Lösungen und Gele, sowie Emulsionen, die eine Ölphase und eine Wasserphase enthalten.

Als flüssige Haut- und Körperpflegeprodukte sind Flüssigseifen, Shampoos, Duschprodukte, sowie Cremen, Gele und dergleichen seit langem bekannt wie auch der Einsatz derartiger Emulsionen zur Haut- und Körperpflege. Beispielsweise wurden Rezepturen für solche Produkte sowohl in der Fachliteratur als auch durch Rohstoffhersteller veröffentlicht. Stellvertretend für die umfangreiche Fachliteratur wird hier auf das "Kosmetikjahrbuch 1992", VCI Verlag für Chemische Industrie, Augsburg, verwiesen. Zu allen nachstehend zitierten Stoffen und deren INCI-Namen (INCI = "International Nomenclature Cosmetic Ingredient") vgl. beispielsweise in "International Cosmetic Ingredient Dictionary" (The Cosmetic, Toiletry, and Fragrance Association, 1993).

Emulsionen sind aus drei Teilen aufgebaut: Sie enthalten eine Ölphase, eine Wasserphase sowie Emulgatoren, um beide Phasen stabil miteinander zu verbinden. Weiterhin ist es Stand der Technik, in die Ölphase öllösliche Wirkstoffe, wie z.B. Vitamine, einzuarbeiten und in der Wasserphase wasserlösliche Produkte, wie z.B. Allantoin, Aminosäuren, Salze wie Natriumchlorid oder Magnesiumsulfat oder Glycerin, Sorbit, Propylenglykol usw., einzusetzen.

Zur Einsatzkonzentration dieser Produkte gibt es keine aus dem Stand der Technik ableitbaren allgemeinen Richtlinien.

Aus FR-A-2688133 ist eine Meersalzlösung mit weiteren Zusätzen (Al, Ag, Cl, Cr, Co, Cu, Fe, F, Ge, Li, etc.) in kolloidaler, metallischer Form, als organische oder mineralische Salze, oder als Komplexe organometallischer bzw. metalloproteinischer Art, zur Behandlung der Atemwege, der Mundhöhle, der Haut und gynäkologischer Schleimhäute bekannt. Das Meerwasser kann bis auf einen Drittel der (zum Blut) isotonischen Konzentration verdünnt werden und mittels eines Puffers auf dem pH-Wert 7 gehalten werden.

Es ist auch bekannt, in den Produkten der eingangs genannten Art Tenside oder Gemische davon einzusetzen, beispielsweise Natriumlaurylsulfat (CAS-Nr. 151-21-3, INCI-Namen "Sodium Lauryl Sulfate"), Natriumpolyoxyethylenlaurylsulfat (CAS-Nr. 9004-82-4, INCI-Namen "Sodium Laureth Sulfate"), Natriumpolyoxyethylen(6)kokosfettsäureamidcarboxylat (INCI-Namen "Sodium PEG-6 cocamide carboxylate"), Natriumpolyoxyethylen(11)laurylethercarboxylat (CAS-Nr. 33939-64-9, INCI-Namen "Sodium Laureth-11 Carboxylate"), Kokosfettsäureamidopropylbetain (CAS-Nr. 61789-40-0; 83138-08-3; 86438-79-1, INCI-Namen "Cocamidopropyl Betaine") usw. und/oder deren Gemische. Die Konzentrationen der Tenside können dabei in weiten Bereichen variieren.

Neben den Tensiden und/oder Gemischen davon können in fertigen Produkten der eingangs genannten Art auch Verdickungsmittel, Rückfetter, Hautschutzstoffe, Parfüms usw. wie auch gegebenenfalls Konservierungsmittel, Farbstoffe usw. enthalten sein.

Es ist aus der Literatur bekannt, dass die üblicherweise als Basistenside eingesetzten Natriumlaurylsulfat und Natriumpolyoxyethylenlaurylsulfat in ihrer Verträglichkeit durch Verschnitt mit den anderen vorangehend genannten Tensiden verbessert werden. Zur objektiven Prüfung der Verträglichkeit von Tensiden und damit hergestellten fertigen Produkten der genannten Art sind eine Vielzahl von Tests vorgeschlagen worden, beispielsweise der Duhring-Kammer-Test, der Patch-Test, die Hautoberflächenfotografie, die Profilometrie, der Methylenblau-Test, der transepidermale Wasserverlust und dergleichen mehr.

Zusätzlich zu solchen Testmethoden ist jedoch die Prüfung des Hautgefühls nach der Anwendung der Produkte der eingangs genannten Art von besonderer Bedeutung, da das Hautgefühl für den Konsumenten kaufentscheidend ist. Dabei kann das Hautgefühl durchaus von den Ergebnissen der vorher erwähnten Tests abweichen. Beispielsweise hat Schrader in "Parfümerie und Kosmetik", 75. Jahrgang, Nr. 2/94, über sehr gute Verträglichkeitstests bei Natriumpolyoxyethylen(3)laurylsulfosuccinat (INCI-Namen "Natrium Laureth-3 Sulfosuccinate") berichtet, während dieses Tensid bei der Prüfung des Hautgefühls deutlich schlechter abschnitt, als nach den übrigen Testmethoden zu erwarten war.

Bei der Entwicklung eines Produkts der eingangs genannten Art genügt es also nicht, die bekannten Testmethoden anzuwenden, um sicherzustellen, dass das fertige Produkt dem Anwender auch ein angenehmes Hautgefühl bringen wird.

Aufgabe der Erfindung ist es deshalb, bei einem Produkt der eingangs genannten Art einen Bezug zwischen der Zusammensetzung des Produkts und einem angenehmen Hautgefühl beim Anwender des Produkts und somit eine Lehre zur Entwicklung und Herstellung eines dem Anwender möglichst angenehmen Produkts der eingangs genannten Art vorzuschlagen.

Zur Lösung dieser Aufgabe sind flüssige Haut- und Körperpflegeprodukte erfindungsgemäss dadurch gekennzeichnet, dass sie im anwendungsfertigen Zustand isotonisch und - bezüglich der Kalium-Ionen und/oder der Magnesium-Ionen - isoionisch zum menschlichen Serum eingestellt sind.

Bevorzugte Ausführungsbeispiele der erfindungsgemässen flüssigen Haut- und Körperpflegeprodukte sind Flüssigseifen, Shampoos und Duschprodukte, sowie Emulsionen, Cremen und Gele. Diese Aufzählung ist jedoch nicht abschliessend, es kommen durchaus auch andere denkbare Ausführungsbeispiele in Betracht.

Es wurde nämlich bei der Prüfung des Hautgefühls verschiedener experimenteller Fertigprodukte der eingangs genannten Art in einer Versuchsreihe überraschenderweise gefunden, dass die isotonisch zum menschlichen Serum eingestellten Fertigprodukte von den Probanden bevorzugt wurden. In einer weiteren Versuchsreihe wurde wiederum überraschenderweise gefunden, dass unter den isotonisch zum menschlichen Serum eingestellten Fertigprodukten die darüber hinaus auch bezüglich der Kalium-Ionen und/oder der Magnesium-Ionen isoionisch zum menschlichen Serum eingestellten Fertigprodukte von den Probanden bevorzugt wurden.

Im nachstehenden werden Ausbildungsbeispiele der Erfindung näher beschrieben.

### Beispiel 1

Zur Herstellung eines erfindungsgemässen Shampoos wird ein Produkt mit folgender Zusammensetzung bereitgestellt:

| | |
|---|---|
| Magnesiumpolyoxyethylen(3)kokosfettsäureamidsulfat (INCI-Namen "Magnesium PEG-3 Cocamide Sulfate") | 6,0 % |
| Kokosfettsäureamidopropylbetain (CAS-Nr. 61789-40-0; 83138-08-3; 86438-79-1, INCI-Namen "Cocamidopropyl Betaine") | 2,5 % |
| Hydrierte Talgfettsäureglyceride (CAS-Nr. 61789-09-1, INCI-Namen "Hydrogenated Tallow Glyceride") | 2,0 % |
| Kaliumsalz von Kokosfettsäure-Kollagen-Kondensat (CAS-Nr. 68920-65-0, INCI-Namen "Potassium Cocoyl Hydrolyzed Collagen") | 0,5 % |
| Phenoxyethanol (CAS-Nr. 122-99-6) | 0,5 % |
| Kaliumsorbat | 0,2 % |
| Parfüm | 0,5 % |
| Guar-2-Hydroxypropylether (CAS-Nr. 39421-75-5, INCI-Namen "Hydroxyropylguar") | 1,0 % |
| Citronensäure | ad pH 5,5 |
| Wasser | ad 100 % |

Zur Herstellung des erfindungsgemässen Shampoos wird dann bei dem so zusammengesetzten Produkt der isotonische osmotische Druck mit Natriumchlorid eingestellt.

Von Testpersonen wird, in bezug auf das Hautgefühl, das isotonisch zum menschlichen Serum eingestellte Produkt dem nicht isotonisch eingestellten Produkt deutlich vorgezogen.

### Beispiel 2

Zur Herstellung eines erfindungsgemässen Duschprodukts wird ein Produkt mit folgender Zusammensetzung bereitgestellt:

| | |
|---|---|
| Natriumpolyoxethylen(11)laurylethercarboxylat (CAS-Nr. 33939-64-9, INCI-Namen "Sodium Laureth-11 Carboxylate'') | 4,0 % |
| Kokosfettsäureamidopropylbetain (CAS-Nr. 61789-40-0; 83138-08-3; 86438-79-1, INCI-Namen "Cocamidopropyl Betaine") | 2,0 % |
| Hydrierte Talgfettsäureglyceride (CAS-Nr. 61789-09-1, INCI-Namen "Hydrogenated Tallow Glyceride") | 1,6 % |
| Kaliumsalz von Kokosfettsäure-Kollagen-Kondensat (CAS-Nr. 68920-65-0, INCI-Namen "Potassium Cocoyl Hydrolyzed Collagen") | 0,4 % |
| Phenoxyethanol (CAS-Nr. 122-99-6) | 0,5 % |
| Sorbinsäure | 0,15 % |
| Parfüm | 0,5 % |
| Kaliumsulfat | 0,039 % |
| Magnesiumsulfat | 0,0045 % |
| Guar-2-Hydroxypropylether (CAS-Nr. 39421-75-5, INCI-Namen "Hydroxypropylguar") INCI-Namen "Hydroxypropylguar") | 1,0 % |
| Citronensäure | ad pH 5,5 |
| Wasser | ad 100 % |

Zur Herstellung des erfindungsgemässen Duschprodukts wird dann bei dem so zusammengesetzten Produkt der isotonische osmotische Druck mit Natriumchlorid eingestellt.

Von Testpersonen wird, in bezug auf das Hautgefühl, das bezüglich der Kalium-Ionen und der Magnesium-Ionen isoionisch zum menschlichen Serum eingestellte Produkt dem analogen, jedoch kein Kaliumsulfat und Magnesiumsulfat enthaltenden d.h. nicht isoionisch eingestellten Produkt deutlich vorgezogen.

Auch wird von Testpersonen, in bezug auf das Hautgefühl, das sowohl bezüglich der Kalium-Ionen wie auch bezüglich der Magnesium-Ionen isoionisch zum menschlichen Serum eingestellte Produkt dem analogen, jedoch nur bezüglich der Kalium-Ionen bzw. nur bezüglich der Magnesium-Ionen isoionisch eingestellten Produkt deutlich vorgezogen.

### Beispiel 3

Zur Herstellung einer erfindungsgemässen Flüssigseife wird ein Produkt mit folgender Zusammensetzung bereitgestellt:

| | |
|---|---|
| Magnesiumpolyoxyethylen(3)kokosfettsäureamidsulfat (INCI-Namen "Magnesium PEG-3 Cocamide Sulfate") | 4,0 % |
| Natriumpolyoxyethylen(11)laurylethercarboxylat (CAS-Nr.33939-64-9, INCI-Namen "Sodium Laureth-11 Carboxylate") | 1,5 % |
| Kokosfettsäureamidopropylbetain (CAS-Nr. 61789-40-0; 83138-08-3; 86438-79-1, INCI-Namen "Cocamidopropyl Betaine") | 1,0 % |
| Hydrierte Talgfettsäureglyceride (CAS-Nr. 61789-09-1, INCI-Namen "Hydrogenated Tallow Glyceride") | 2,0 % |
| Kaliumsalz von Kokosfettsäure-Kollagen-Kondensat (CAS-Nr. 68920-65-0, INCI-Namen "Potassium Cocyl Hydrolyzed Collagen") | 0,5 % |
| Polymeres Quaterniumsalz von Methylvinylimidazoliumchlorid und Vinylpyrrolidon (CAS-Nr. 29297-55-0, INCI-Namen "Polyquaternium-16") | 0,5 % |
| Benzylalkohol | 0,3 % |
| Sobinsäure | 0,1 % |
| Parfüm | 0,1 % |
| Guar-2-Hydroxypropylether (CAS-Nr. 39421-75-5, INCI-Namen "Hydroxypropylguar") | 0,7 % |
| Citronensäure | ad pH 5,5 |
| Wasser | ad 100 % |

Zur Herstellung der erfindungsgemässen Flüssigseife wird bei dem so zusammengesetzten Produkt der isotonische osmotische Druck mit Natriumchlorid eingestellt.

Von Testpersonen wird, in bezug auf das Hautgefühl, das isotonisch zum menschlichen Serum eingestellte Produkt dem nicht isotonisch eingestellten Produkt deutlich vorgezogen.

### Beispiel 4

Zur Herstellung einer erfindungsgemässen Emulsion wird ein Produkt mit folgender Zusammensetzung bereitgestellt:

| | |
|---|---|
| Polyglyceryl-2-PEG-4-stearat | 6,0 % |
| Lanolinalkohol | 1,5 % |
| Triceteareth-4-phosphat | 1,5 % |
| Mineralöl | 3,0 % |
| Isopropylpalmitat | 4,0 % |
| Caprylsäure/Caprinsäuretriglyceride | 2,0 % |
| Tocopherolacetat | 1,0 % |
| Panthenol | 1,0 % |
| Allantoin | 0,2 % |
| Carbomer | 0,25 % |
| Zitronensäure | 0,1 % |
| Kaliumsorbat | 0,2 % |
| Natriumhydroxymethylglycinat | 0,1 % |
| Duftstoff | 0,2 % |
| Wasser | ad 100 % |

Diese Emulsion hatte eine Osmolarität von 150 mosmol/kg. Diese Emulsion wurde dann mit jedem der folgenden Rohstoffe auf einen osmotischen Druck von 600 mosmol/kg und 290 bis 300 mosmol/kg (osmotischer Druck des menschlichen Serums) eingestellt:
1) Natriumchlorid
2) Glycerin
3) Sorbit
4) 1,2-Propylenglykol

In einem Konsumententest wurden diese verschiedenen Produkte bezüglich der Akzeptanz durch Kunden geprüft. Dabei stellte sich überraschenderweise heraus, dass die isotonisch zum menschlichen Serum eingestellten Produkte von den Konsumenten im Vergleich zum hypotonischen Produkt und den hypertonischen Produkten bevorzugt wurden.

## Patentansprüche

1. Flüssige Haut- und Körperpflegeprodukte **dadurch gekennzeichnet**, dass sie im anwendungsfertigen Zustand isotonisch und - bezüglich der Kalium-Ionen und/oder Magnesium-Ionen - isoionisch zum menschlichen Serum eingestellt sind.

2. Flüssige Haut- und Körperpflegeprodukte nach Anspruch 1, **dadurch gekennzeichnet, dass** sie eine Öl-in-Wasser Emulsion darstellen.

3. Flüssige Haut- und Körperpflegeprodukte nach einem der Ansprüche 1 oder 2, **dadurch gekennzeichnet, dass** sie als Flüssigseifen ausgebildet sind.

4. Flüssige Haut- und Körperpflegeprodukte nach einem der Ansprüche 1 oder 2, **dadurch gekennzeichnet, dass** sie als Shampoos ausgebildet sind.

5. Flüssige Haut- und Körperpflegeprodukte nach einem der Ansprüche 1 oder 2, **dadurch gekennzeichnet, dass** sie als Duschprodukte ausgebildet sind.

6. Flüssige Haut- und Körperpflegeprodukte nach einem der Ansprüche 1 oder 2, **dadurch gekennzeichnet, dass** sie als Cremen oder Gele ausgebildet sind.

## Claims

1. Liquid skin- and bodycare products, characterized in that, in the ready-to-use state, they have been made isotonic and - with respect to the potassium ions and/or magnesium ions - isoionic to human serum.

2. Liquid skin- and bodycare products according to Claim 1, characterized in that they are an oil-in-water emulsion.

3. Liquid skin- and bodycare products according to one of Claims 1 or 2, characterized in that they are in the form of liquid soaps.

4. Liquid skin- and bodycare products according to one of Claims 1 or 2, characterized in that they are in the form of shampoos.

5. Liquid skin- and bodycare products according to one of Claims 1 or 2, characterized in that they are in the form of shower products.

6. Liquid skin- and bodycare products according to one of Claims 1 or 2, characterized in that they are in the form of creams or gels.

## Revendications

1. Produits liquides pour les soins de la peau et du corps, caractérisés en ce qu'ils sont soumis, à l'état prêt à l'emploi, à un réglage isotonique et - en ce qui concerne les ions potassium et/ou les ions magnésium - à un réglage isoionique par rapport au sérum humain.

2. Produits liquides pour les soins de la peau et du corps selon la revendication 1, caractérisés en ce qu'ils représentent une émulsion du type huiledans-l'eau.

3. Produits liquides pour les soins de la peau et du corps selon l'une quelconque des revendications 1 ou 2, caractérisés en ce qu'ils sont réalisés sous forme de savons liquides.

4. Produits liquides pour les soins de la peau et du corps selon l'une quelconque des revendications 1 ou 2, caractérisés en ce qu'ils sont réalisés sous forme de shampooings.

5. Produits liquides pour les soins de la peau et du corps selon l'une quelconque des revendications 1 ou 2, caractérisés en ce qu'ils sont réalisés sous forme de produits pour la douche.

6. Produits liquides pour les soins de la peau et du corps selon l'une quelconque des revendications 1 ou 2, caractérisés en ce qu'ils sont réalisés sous forme de crèmes ou de gels.
